Europäisches Patentamt

(19) European Patent Office

Office européen des brevets

(11) Publication number: **0 277 770**
**A1**

## (12) EUROPEAN PATENT APPLICATION

(21) Application number: **88300721.3**

(22) Date of filing: **28.01.88**

(51) Int. Cl.⁴: **C 12 N 15/00**
C 12 N 1/16, C 12 P 21/00
//(C12P21/00,C12R1:85),
(C12P21/00,C12R1:865)

(30) Priority: **02.02.87 US 10107**

(43) Date of publication of application:
**10.08.88 Bulletin 88/32**

(84) Designated Contracting States:
**CH DE FR GB IT LI NL**

(71) Applicant: **MERCK & CO. INC.**
**126, East Lincoln Avenue P.O. Box 2000**
**Rahway New Jersey 07065-0900 (US)**

(72) Inventor: **Ellis, Ronald W.**
**1407 Edgeville Road**
**Overbrook Hills Pennsylvania 19151 (US)**

**Schultz, Loren D.**
**421 Oak Drive**
**Harleysville Pennsylvania 19438 (US)**

**Montgomery, Donna L.**
**9 Hickory Lane**
**Chalfont Pennsylvania 18914 (US)**

**Markus, Henry Z.**
**1517 Thornberry Road**
**Wyncote Pennsylvania 19015 (US)**

(74) Representative: **Hesketh, Alan, Dr.**
**European Patent Department Merck & Co., Inc. Terlings**
**Park Eastwick Road**
**Harlow Essex, CM20 2QR (GB)**

(54) **Alpha-mating factor promoter is modified by deleting pre-pro secretory leader sequence.**

(57) A novel vector has been developed which enables foreign genes to be expressed intracellularly in yeast in a regulatable expression system. By means of this vector, the complete envelope gene of hepatitis B virus has been expressed in Saccharomyces cerevisiae. The expressed protein aggregates into a particulate form which displays the major antigenic sites of the protein, thereby highlighting the utility of this vector to direct expression of foreign genes in yeast.

Fig. 1

EP 0 277 770 A1

**Description**

## ALPHA-MATING FACTOR PROMOTER IS MODIFIED BY DELETING PRE-PRO SECRETORY LEADER SEQUENCE

BACKGROUND OF THE INVENTION

Recombinant DNA technology has made possible the introduction into intracellular host environments of a variety of functional genes that are foreign or heterologous to the host cell. Classically, the first genes to be recombined involved ribosomal DNA or structural genes of eukaryotic origin in a procaryotic host. When a heterologous DNA is translated in the host, an expression system results.

Yeast cells have provided a variety of eukaryotic expression systems. They have many advantages over procaryotic expression systems for the expression of foreign or heterologous eukoryotic DNA sequences, since yeast are eukaryotic in nature. For example, glycosylation and other forms of post-translational modification in yeast are closer to those performed by the original cell from which the heterologous eukaryotic DNA sequence was derived. However, one well-known difference between yeast cells and those of higher eukaryotes is the substantially complete absence of intervening sequences in the structural genes of yeast such as Saccharomyces cerevisiae.

One of the yeast expression systems of utility to the commercial production of heterologous proteins utilizes the promoter from the yeast MFα1 gene, which encodes a yeast mating factor referred to as α factor. Yeast has 2 mating types: a and α. The mating type is determined by the information at the MAT locus on Chromosome III. If the α sequence is present, the cell is an α type. If the a sequence is present, the cell is an a type. In a diploid strain, if the α sequence is present at one MAT locus and the a sequence present at the other MAT locus, the strain is an a/α type. Mating occurs between a and α cells, neither of which can sporulate. Sporulation occurs in a/α cells, but they are not able to mate.

Silent copies of both the a and α sequences also are present on Chromosome III. Cells containing the HO (homothallic) gene are able to switch their mating type by transferring the information from the silent copies to the MAT locus. In contrast, ho (heterothallic) strains are unable to transfer this information and therfore can maintain stable mating types. See, e.g., I. Herskowitz et al., "Control of Cell Type in Saccharomyces cerevisiae: Mating Type and Mating-Type Interconversion," in Strathern, J.N. et al., (eds.) Molecular Biology of The Yeast Saccharomyces, Cold Spring Harbor 1981, pp. 181 et seq., and K.A. Nasmyth Ann. Rev. Genet. 16, 439 (1982).

The α-factor in yeast is a 13 amino acid peptide secreted by cells of the α mating type. It acts specifically on a haploids to cause arrest in the G1 phase of the cell-cycle. The alpha mating factor (MFα1) promoter expression system has been used by others to direct the expression and secretion of foreign proteins in yeast. In contrast, the present inventors have used the same promoter to obtain non-secretory products by substantially modifying the MFα1 promoter expression system to provide expression of non-secreted heterologous proteins, with the object inter alia of obtaining better products for purposes of vaccination.

The present inventors are the first to use the MFα1 promoter to express a heterologous protein intracellularly without secretion. Several groups have utilized a vector containing the MFα1 promoter to express foreign genes in S. cerevisiae. Such vectors contain the promoter and pre-pro-leader sequence for the secretion of polypeptides. The leader sequence directs the primary translational product to the rough endoplasmic reticulum for traversal of the secretory pathway. For example, Kurjan, J. et al., U.S. Patent 4,546,082, use secretory sequences in their α-factor constructions. Similarly, α-factor constructions utilizing secretory sequences are reported in Brake, A.J. et al., Proc. Natl. Acad. Sci. 81, 4642 (August 1984); Singh, A. et al., Nucl. Acids Res. 12, 8927 (1984); Bitter, G. A. et al., Proc. Natl. Acad. Sci. 81, 5330 (September 1984); Emr, S.D. et al., Proc. Natl. Acad. Sci. 80, 7080 (December 1983); Gardell, S.J. et al., Nature 317, 551 (October 1985); Miyajima, A. et al., Gene 37, 155 (1985) and Mullenbach, G. T. et al., J. Biol. Chem. 261, 719 (1986).

The present inventors have deleted the DNA sequences which encode secretory signal sequences of α-factor protein and have constructed an α-factor promoter sequence directly fused to the structural sequence of a heterologous protein. The unobvious result was intracellular expression of the heterologous protein without detectable degradation and without secretion. In one case, the complete envelope gene of hepatitis B virus was expressed in Saccharomyces cerevisiae. The expressed protein was found to aggregate into particulate form and to display the major antigenic sites of the protein, thereby highlighting the utility of the invention.

One principal advantage of the present invention is the use of the α-factor promoter expression system to express without secretion a protein having a normally intracellular locale. Therefore, synthesis of an intracellular eukaryotic protein with the present expression system will result in a protein with modifications and conformations more closely resembling the native protein as synthesized in its final form in its native or natural cell. Such considerations are critical to enhancing the immunogenicity of the protein.

The present invention also is directed to a method for the expression in yeast of foreign genes whose primary translational product might be toxic to the host cell and is normally disposed intracellularly, not extra-cellularly. In addition, the present invention is directed to regulatable expression of proteins under growth conditions in which there would not be a change in cellular physiology of a type effected by a change in the composition of the growth medium, e.g., carbon source. Such a change is detrimental to certain aspects of the expression of a foreign gene. It will be apparent to those skilled in the art that the principle of expressing the complete envelope protein of hepatitis B virus (HBV) intracellularly by means of the novel vector extends to other foreign genes whose protein product normally is expressed intracellularly.

It is an object of the present invention to provide expression vectors for the regulatable expression of foreign genes in yeast. Another object is to provide a vector for the expression of the complete envelope gene of (HBV) in a fashion which does not depend upon a change in cell physiology resulting from a change in growth medium. These and other objects of the present invention will be apparent from the description given below.

## BRIEF DESCRIPTION OF THE INVENTION

An expression system is disclosed for the intracellular expression of heterologous proteins comprising yeast cells transformed by an expression vector, said vector comprising the DNA sequence for the promoter of the alpha mating factor of yeast joined directly without secretory sequences to a structural gene of an origin foreign to the yeast, said structural gene expressed in the yeast cell without secretion. One such structural gene is the envelope protein of HBV.

In this expression system, the inventors of the present application have shown expression of the envelope protein of HBV. It is apparent that other heterologous gene sequences, or portions thereof, can be expressed in this expression system.

## BRIEF DESCRIPTION OF THE DRAWINGS

Figure 1 is a schematic diagram illustrating the construction of plasmid pYGAL/PSSC.
Figure 2 is a schematic diagram illustrating the construction of plasmid pRJ178.
Figure 3 is a schematic diagram illustrating the construction of plasmid pJC197.
Figure 4 is a schematic diagram illustrating the construction of plasmid pBRαF-1.
Figure 5 is a schematic diagram illustrating the construction of plasmid pHM1.
Figure 6 is a schematic diagram illustrating the construction of plasmid pHM1/PSSC.

## ABBREVIATIONS AND DEFINITIONS

HMRa designates the wild-type allele a of the HMR cassette.

HMRα designates the wild-type allele α of the HMR cassette.

HMLa designates the wild-type allele a of the HML cassette.

HMLα designates the wild-type allele α of the HML cassette.

MATa designates the wild-type a allele of the mating locus.

MATα designates the wild-type α allele of the mating locus.

MFα1 designates the gene for alpha mating factor.

Regulatable expression: expression of DNA sequences in a system inducible for such expression without a change in the chemical composition of the growth medium outside the cell.

SIR designates the wild-type silent information regulator which represses transcription of silent copy loci of HMR or HML. There are 4 different SIR genes (SIR1, SIR2, SIR3, SIR4).

sir3-8 designates a mutation arbitrarily numbered 8 in the third SIR complementation group. Mutants of SIR have direct expression of mating type information from HML and HMR, which are normally silent.

## DETAILED DESCRIPTION OF THE INVENTION

Hepatitis B virus (HBV) is the infectious agent responsible for several varieties of human liver disease. Many individuals who are infected by HBV suffer through an acute phase of disease, which is followed by recovery. However, a large number of individuals fail to clear their infection, thereby becoming chronic carriers of the infection. HBV infection is endemic to many parts of the world, with a high incidence of infection occurring perinatally from chronically infected mothers to their newborns. The number of chronic carriers worldwide has been estimated at over three hundred million. From this pool of carriers, hundreds of thousands die annually from the long-term consequences (cirrhosis or hepatocellular carcinoma) of chronic hepatitis B infection.

The HB virion is composed of two groups of structural proteins, the core proteins and the envelope or surface ("S") proteins. In addition to being the major surface proteins of the virion, i.e., Dane particle, the "S" proteins are the sole constituents of Australia antigen, or 22 nm particles. The "S" proteins are the translational products of a large open reading frame (ORF) encoding 389-400 amino acids, depending upon subtype. This ORF is demarcated into three domains, each of which begins with an ATG codon that is capable of functioning as a translational initiation site in vivo. These domains are referred to as preS1 (108-119 amino acids), preS2 (55 amino acids), and S (226 amino acids) in their respective 5'-3' order in the gene. The six protein products

3

derived from this ORF have the following compositions:

1) gp42 (42,000 dalton glycoprotein) = preS1/preS2/S (meaning preS1, contiguous with preS2, contiguous with S) with glycosylation,

2) p39 (p = protein) = preS1/preS2/S, also known as the complete envelope protein; no glycosylation

3) gp36 = preS2/S (two glycosylation sites),

4) gp33 = preS2/S (one glycosylation site),

5) gp27 = S (one glycosylation site),

6) p24 = S.

All six proteins are present to an approximately equimolar extent in the HBV Dane particle. In the 22 nm particle, the 4 smaller proteins are present to an approximately equimolar extent, while gp42 and p39 are present in at most one or a few molecules per particle.

The 22 nm particles, or HB surface antigen (HBsAg) particles, have been purified from the plasma of chronic carriers. In terms of their plasma being particle-positive, these chronic carriers are referred to as HBs+. When these carriers have mounted a sufficient immune response, they can clear the infection and become HBs-. In terms of their formation of antibodies to HBs, these individuals are denoted anti-HBs+. In this way, anti-HBs+ is correlated with recovery from disease. Therefore, the stimulation or formation of anti-HBs+ by HB vaccine has been expected to confer protection against HBV infection.

This hypothesis has been testable experimentally. Outside of man, chimpanzees are the only species which is fully susceptible to HBV infection, as reflected in quantifiable markers such as HBs+, elevated serum levels of liver enzymes, etc. Chimpanzees have been vaccinated with three doses of purified HBsAg particles and then challenged with a large dose of infectious HBV. While mock-vaccinated animals have suffered the signs of acute HBV infection, the HBsAg-vaccinated animals have been protected completely from any signs of infection. Therefore, in this experimental system, HBsAg particles, composed of gp27 and p24 (S domain only), have been sufficient to induce protective immunity. Spurred by these observations, several manufacturers have produced HB vaccines composed of HBsAg particles.

Recent data have suggested that the preS1 and preS2 domains may play an important role in immunity to HBV infections. Both antibodies to preS1 (elicited by immunization with a peptide consisting of amino acid residues 10-32 of preS1) as well as antibodies to preS2 (elicited by immunization with a peptide consisting of amino acid residues 1-26 of preS2) are capable of blocking the binding of HBV to human hepatoma cells in vitro; anti-HBs (sera from patients vaccinated with HBsAg lacking preS1 or preS2) is incapable of mediating this blocking event. If this in vitro event mimics in vivo infection, then preS (i.e., preS1 and preS2 in toto linked together) domains may represent the HBV binding site to its liver cell receptor, and anti-preS may block HBV attachment and initiation of infection. In addition, it has been found that anti-preS rises in titer during the recovery phase from acute HBV infection, indicating a role for these antibodies in recovery. Finally, it has been shown that vaccination of chimpanzees with either a 109 amino acid preS1 polypeptide (residues 27-119 of preS1 contiguous with 1-16 of preS2) or a 55 amino acid preS polypeptide (residues 1-55 of preS2) was capable of mediating some measure of protection against HBV challenge. In sum, these experimental observations have suggested that the preS domains are a useful addition to present HB vaccines.

In order to expand the available supply of HB vaccines, manufacturers have turned to recombinant DNA technology to mediate the expression of "S" proteins. Among microbial systems, Escherichia coli and S. cerevisiae have been used most commonly for the expression of many recombinant-derived proteins. Numerous attempts to express immunologically active HBsAg particles in E. coli have been unsuccessful. However, S. cerevisiae has shown great versatility in its ability to express immunologically active HBsAg particles. These particles, when formulated into a vaccine, have proven capable of fully protecting chimpanzees against challenge with live HBV. Furthermore, yeast-derived HBsAg (licensed as Recombivax HB,® Merck, Sharp and Dohme) has been as effective immunologically in human clinical trials as plasma-derived HBsAg. Therefore, the utility of S. cerevisiae as a host species for directing synthesis of recombinant HBsAg is established firmly. In light of this, it would be desirable to express the entire preS domain linked to the S domain in an immunogenic form.

In a variety of recombinant microbial expression systems, the synthesis of many different polypeptides has been shown to be deleterious to the host cell. As a consequence, there is selective pressure against the expression of such polypeptides, such that the only cells which accumulate in a scale-up of such a recombinant culture are those which have ceased to express the foreign polypeptide or express so little of the foreign polypeptide that the culture becomes an uneconomical source of that polypeptide. In some cases, the deleterious effect is so strong that when expression is driven by a strong constitutive promoter, newly transformed cells fail to propagate and form colonies on selective plates. These deleterious effects can be overcome by using an inducible promoter to direct the synthesis of such polypeptides. A number of inducible genes exist in S. cerevisiae. Three well-characterized inducible systems are the galactose (GAL) utilization genes, the alcohol dehydrogenase 2 (ADH2) gene, and the α mating factor gene.

S. cerevisiae has 3 genes which encode the enzymes responsible for the utilization of galactose as a carbon source for growth. The GAL1, GAL7 and GAL10 genes respectively encode galactokinase, α-D-galactose-1-phosphate uridyltransferase and uridine diphosphogalactose-4-epimerase. In the absence of galactose, very little expression of these enzymes is detected. If cells are grown on glucose and then galactose is added to the culture, these three enzymes are induced coordinately, by at least 1,000-fold, at the level of RNA transcription. The GAL1 and GAL10 genes have been cloned and sequenced. The regulatory and promoter sequences to the

5' sides of the respective coding regions have been placed adjacent to the coding regions of the lacZ gene. These experiments have defined those promoter and regulatory sequences which are necessary and sufficient for galactose induction.

S. cerevisiae also has 3 genes, each of which encodes an isozyme of alcohol dehydrogenase (ADH). One of these enzymes, ADHII, is responsible for the ability of S. cerevisiae to utilize ethanol as a carbon source during oxidative growth. Expression of the ADH2 gene, which encodes the ADHII isozyme, is catabolite-repressed by glucose, such that there is virtually no transcription of the ADH2 gene during fermentative growth in the presence of glucose levels of >0.1% (w/v). Upon glucose depletion and in the presence of non-repressing carbon sources, transcription of the ADH2 gene is induced 100- to 1000-fold. This gene has been cloned and sequenced, and those regulatory and promoter sequences which are necessary and sufficient for derepression of transcription have been defined.

Alpha mating factor ($\alpha$ mating factor) is a sex pheromone of S. cerevisiae which is required for mating between MAT$\alpha$ and MAT$\overline{a}$ cells. This tridecapeptide is expressed as a prepropheromone which is directed into the rough endoplasmic reticulum, glycosylated and proteolytically processed to its final mature form, then secreted from cells. This biochemical pathway has been exploited as an expression strategy for the secretion of foreign or heterologous polypeptides. The alpha mating factor gene has been cloned and its promoter with pre-pro- leader sequence has been utilized to express and secrete a variety of polypeptides. As expected by their traversal of the rough endoplasmic reticulum and Golgi apparatus, foreign proteins can undergo both N- and 0-linked glycosylation events.

The alpha mating factor promoter is active only in cells which are phenotypically $\alpha$. There are 4 genetic complementation groups in S. cerevisiae known as silent information regulator or SIR, which are required for the repression of the expression of a and $\alpha$ information normally present at the HML and HMR loci respectively. Temperature-sensitive (ts) lesions which interfere with this repression event exist in several of these complementation groups. In one mutant, designated sir3-8, growth at 35°C abrogates repression, resulting in cells becoming phenotypically a/$\alpha$ in which the alpha mating factor promoter is inactive. Upon temperature shift to 23°C, the cells phenotypically revert to $\alpha$, and the promoter becomes active. The use of strains with a ts SIR lesion has been attempted for the controlled expression of several foreign secreted polypeptides.

Dane particles are utilized as the source of HBV nucleic acid for the isolation of the preS1/preS2/S ORF. The endogenous polymerase reaction is employed in order to produce covalently-closed circular double-stranded DNA of the HBV genome from the nicked and gapped form that resides natively in the HB virion. The repaired DNA is isolated and digested to completion with EcoRI. The E. coli cloning vector pBR322 also is digested with EcoRI, ligated to the HBV DNA and used to transform E. coli. Recombinant plasmids are selected, i.e., those containing the HBV genome in a circularly permuted form in which the EcoRI site divides the complete preS1/preS2/S coding region into a 5' domain of 0.4 kilobase pairs (kbp) and a 3' domain of 0.8 kbp. These two domains are subcloned for the eventual reassembly of the entire gene. For the 3' domain, pUC19 is digested with EcoRI and BamHI, then ligated to a synthetic oligonucleotide which consists of the final 5 nucleotides of the coding region, the stop codon, a HindIII site, and a BamHI end. The 3' portion of the preS1/preS2/S gene, consisting of a 0.8 kbp EcoRI-AccI fragment, is cloned into this vector. For the 5' portion, pUC18 is digested with HindIII and EcoRI and ligated to a 72 bp synthetic oligonucleotide which reconstitutes the complete ORF from the BamHI site upstream, through the distal ATG and a 10 bp nontranslated leader sequence, to a HindIII compatible terminus. The 0.3 kbp BamHI-EcoRI fragment of the 5' domain then is ligated into this oligonucleotide-linked cloning vector. The 5' pUC18 and 3' pUC19 clones are amplified by growth in E. coli, and the coding regions are digested from the isolated plasmids as HindIII-EcoRI fragments. The 5' and 3' fragments are ligated, digested with HindIII, and the complete ORF with HindIII termini is cloned into YEp52 which has been digested previously with HindIII, yielding pYGAL/PSSC. The complete ORF as a HindIII fragment is purified by preparative agarose gel electrophoresis for cloning into the MF$\alpha$1 promoter expression systems.

The alpha mating factor gene MF$\alpha$1 has been cloned onto a plasmid vector from S. cerevisiae genomic DNA. The resulting plasmid pKH2 is digested with EcoRI and the 1.5 kbp fragment bearing the alpha mating factor gene is purified by preparative agarose gel electrophoresis. Plasmid pRJ148 (a modified pBR322 lacking the HindIII site) is digested with EcoRI and ligated with the 1.5 kbp fragment to yield the plasmid pRJ159. This DNA is digested with HindIII and self-ligated to form plasmid pRJ167, which now has a unique HindIII site. Plasmid pRJ167 is digested with HindIII and modified by the insertion of a synthetic oligonucleotide adaptor, which encodes translational termination codons in all three reading frames, to yield a new plasmid (pRJ178) containing a unique HindIII site which is to the 3' side of the promoter and pre-pro-leader and to the 5' side of translational termination signals in all three reading frames. The HindIII site is converted to a BamHI site by digestion with HindIII, flush-ending with the Klenow fragment of DNA polymerase I, addition of BamHI linkers and self-ligation to form plasmid pJC193. This plasmid is digested with EcoRI, flush-ended with the Klenow fragment of DNA polymerase I, modified by the addition of BclI linkers, digested with BclI, and the 1.5 kbp fragment bearing the alpha mating factor gene isolated by preparative agarose gel electrophoresis. This resulting BclI fragment is treated with calf intestine alkaline phosphatase and then is inserted into the unique BamHI site of pCI/1, destroying the original BamHI site in the process (plasmid pJC194). This DNA is digested with BamHI and self-ligated to remove excess BamHI linkers, resulting in the new alpha mating factor expression plasmid pJC197.

Plasmid pJC197 containing the α mating factor gene is digested with PstI. The 0.8 kbp fragment containing a portion of the MFα1 promoter and pre-pro-leader sequences is purified by preparative agarose gel electrophoresis. It then is digested with Bal31 under conditions where 30-40 bp are removed from each end. The DNA is blunt-ended with T4 DNA polymerase and ligated to a synthetic BamHI octameric linker.

The DNA is digested with BamHI and cloned into the BamHI site of pBR322, forming vector pBRαF; transformants of E. coli strain HB101 are screened with two synthetic oligonucleotides that had been end-labelled with γ [$^{32}$P]ATP in the presence of T4 polynucleotide kinase: one oligonucleotide derived from the 5′ noncoding sequence immediately flanking the coding sequence, the other derived from immediate 5′ end of the coding sequence.

Colonies which hybridize only with the former synthetic oligonucleotide are selected for further analysis. DNA is extracted from large-scale cultures of 3 such colonies, digested with BamHI, labelled with α [$^{32}$P]dGTP in the presence of the Klenow fragment of DNA polymerase I, and digested with AvaII. DNA sized 0.25 kbp is purified by preparative gel electrophoresis and sequenced by the Maxam-Gilbert method to verify the absence of the 5′ pre-pro-leader region of the coding sequence. From a candidate clone, pBRαF-1, a larger amount of the 0.25 kbp BamHI - AvaII fragment is purified by preparative gel electrophoresis. The 1.5 kbp fragment of EcoRI-digested pJC197 is purified by preparative gel electrophoresis and cloned into the EcoRI site of pUC7. Transformants are isolated and used to prepare pUCαF-1. Another sample of plasmid pJC197 is digested with PstI and the 0.8 kbp MFα1 promoter fragment is isolated. This fragment then is digested with AvaII, and the resulting 0.6 kbp fragment is purified by preparative gel electrophoresis. pUCαF-1 is digested with PstI and BamHI, the 3.2 kbp vector fragment is purified by preparative agarose gel electrophoresis, then ligated to the previously isolated 0.25 and 0.6 kbp fragments of the MFα1 promoter. The DNA is used to transform E. coli. The resulting vector pUCαF-2 is digested with EcoRI. The 1.2 kbp fragment is blunt-ended with the Klenow fragment of DNA polymerase I, purified by preparative agarose gel electrophoresis, and ligated into the blunt-ended BamHI site of pCl/1. The resulting vector, pHM1, has the following salient features:

1) E. coli-derived sequences for the selection (bla gene) and amplification (ori) of the plasmid in E. coli.
2) S. cerevisiae-derived sequences for the selection (LEU2) and amplification (2-micron DNA) of the plasmid in S. cerevisiae.
3) yeast alpha mating factor promoter.
4) a unique BamHI cloning site for the cloning of a foreign polypeptide to be expressed.
5) a translational termination sequence in all three reading frames.
6) a yeast transcriptional termination sequence.

The 1.1 kbp HindIII fragment of pYGAL/PSSC, which is the complete ORF of preS1/preS2/S, is flush-ended with the Klenow fragment of DNA polymerase I and cloned into the flush-ended BamHI site of vector pHM1 resulting in plasmid pHM1/PSSC.

The MFα1 promoter is active only in cells which are phenotypically α. There are 4 loci in S. cerevisiae, known as SIR, which synthesize proteins required for the repression of other normally silent copies of a and α information. Strain JRY188 (MATα, sir3-8, leu2-3, leu2-112, trp1, ura3-52, his4) contain a ts lesion in the SIR3 gene product. As a result, JRY188 cells grown at 35°C are phenotypically a/α and the alpha mating factor promoter is not active; on the other hand, cells grown at 23°C are phenotypically α and thus capable of expressing a gene directed by the MFα1 promoter. The recombinant PreS1/PreS2/S-containing alpha mating factor plasmid pHM1/PSSC is used to transform S. cerevisiae host strain JRY188 and transformed clones are selected. Cells are grown in synthetic selective (leu−) medium at 35°C and when the culture reaches a density of $A_{600} = 0.5$, the incubation temperature is reduced to 23°C. Lysates are prepared, resolved by SDS-PAGE, and blotted to nitrocellulose. A p39 product is found to be specific to PreS1/PreS2/S by virtue of its presence only in transformants and its reactivity with convalescent human HB sera. Furthermore, lysates of transformants, but not wild-type S. cerevisiae, are positive for HBsAg by radioimmunoassay and are positive for preS by virtue of binding to polymerized human albumin, a binding which has been shown to be specific to the preS region.

The genus Saccharomyces is composed of a variety of species. Most commonly used is Saccharomyces cerevisiae, or bakers' yeast, as a host for the recombinant DNA-mediated expression of a variety of foreign polypeptides. However, the distinctions among other species of the genus Saccharomyces are not always well-defined. Many of these species are capable of interbreeding with S. cerevisiae and are likely to possess regulatable promoters which are analogous or identical to promoters in S. cerevisiae, including, but not limited to GAL10, ADH2 or alpha mating factor promoters. Therefore, it will be apparent to those skilled in the art that, for the expression of preS-containing polypeptides, the selection of a host strain extends to other species of the genus Saccharomyces, including but not limited to carlsbergensis, uvarum, rouxii, montanus, kluyveri, elongisporus, norbensis, oviformis, and diastaticus.

Several other yeast genera, such as Hansenula, Candida, Torulopsis, and Pichia, have been shown to contain similar metabolic pathways for the utilization of methanol as a sole carbon source for growth. The gene for alcohol oxidase, an enzyme which participates in this metabolic pathway, has been isolated from Pichia pastoris. The P. pastoris alcohol oxidase promoter has been isolated and shown to be susceptible to methanol induction of expression. Such an inducible promoter is useful for the expression of polypeptides which are negatively selected in yeast. In particular, this promoter has been shown to be active on a plasmid for the inducible expression of the S domain of HBV in P. pastoris in particulate form. This observation highlights the ability of other yeast genera to function as hosts for the recombinant DNA-mediated expression of S polypeptides in immunologically active form. Therefore, it will be apparent to those skilled in the art that, for the expression of preS-containing polypeptides utilizing temperature-regulated promoters, the selection of a host strain extends to species from other genera of yeast from the Families Saccharomycetaceae and Cryptococcaceae, including, but not limited to Pichia, Candida, Hansenula, Torulopsis, Kluyveromyces, and Saccharomycopsis.

Detailed information on many of the techniques used in this invention can be found in Maniatis, T. et al., Molecular Cloning: A Laboratory Manual Cold Spring Harbor Laboratory 1982; Davis, L.G. et. al., Basic Methods in Molecular Biology, Elsevier 1986.

The following examples illustrate the present invention without, however, limiting the same thereto. The disclosure of each reference mentioned in the following examples is hereby incorporated by reference.

## EXAMPLE 1

### Cloning the preS1/preS2/S Gene

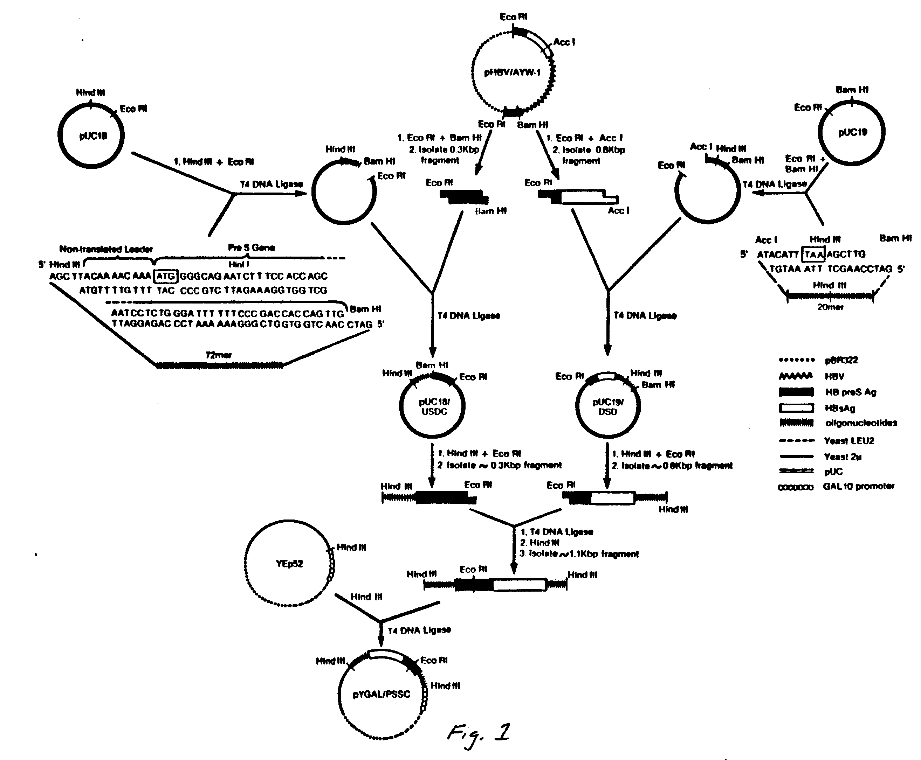

Dane particles (subtype ayw) were purified from the plasma of infected individuals by established techniques [Landers et al., J. Virology 23: 368 (1977)]. The HBV genomic DNA resides in a nicked, gapped form in the virion [Hruska et al., J. Virology 21: 666 (1977)]. In order to prepare this DNA for molecular cloning, the endogenous polymerase reaction was employed to produce covalently closed circular double-stranded DNA [Landers et al., J. Virology 23: 368 (1977)]. The DNA was deproteinized by incubation in buffer containing sodium dodecyl sulfate and Proteinase K, followed by extraction with phenol: chloroform:isoamyl alcohol (25:24:1) and concentration by ethanol precipitation. This purified DNA was digested to completion with EcoRI. The E. coli cloning vector pBR322 also was digested with EcoRI, ligated to the digested HBV DNA and used to transform E. coli. Recombinant plasmids were isolated which contain the HBV genome in a circularly permuted orientation about the unique EcoRI site (pHBV/AYW-1, Figure 1), which divides the complete preS1/preS2/S coding region into a 5′ domain of 0.4 kbp and a 3′ domain of 0.8 kbp [Galibert et al., Nature 281: 646 (1979)] These two domains were subcloned for the eventual reassembly of the entire gene.

For subcloning the 3′ portion of the preS1/preS2/S gene, pUC19 was digested with EcoRI and BamHI, then ligated to a synthetic oligonucleotide which consists of the final 5 nucleotides of the coding region, the stop codon, a HindIII site, and a BamHI end. The structure of this oligonucleotide is

ATACATTTAAAGCTTG
  TGTAAATTTCGAACCTAG.

The 3′ portion of the preS1/preS2/S gene, consisting of a 0.8 kbp EcoRI-AccI fragment was cloned into this vector (pUC19/DSD, Figure 1).

For subcloning the 5′ portion, pUC18 was digested with HindIII and EcoRI and ligated to a 72 bp synthetic oligonucleotide which reconstitutes the complete ORF from the BamHI site upstream to the distal ATG through a 10 bp nontranslated leader sequence to a HindIII compatible terminus. The structure of this oligonucleotide is:

                                                                        \*

AGCTTACAAAACAAAATGGGGCAGAATCTTTCCACCAGCAATCCTCTGGGATTTTT
    ATGTTTTGTTTTACCCCGTCTTAGAAAGGTGGTCGTTAGGAGACCCTAAAAA

TCCCGACCACCAGTTG
AGGGCTGGTGGTCAACCTAG.

(\*the natural sequence contains C rather than T at this position. The above change destroys the HinfI site without changing the encoded amino acid.)

The 0.3 kbp BamHI-EcoRI fragment of the 5′ domain then was ligated into the oligonucleotide-linked cloning vector (pUC18/USDC, Figure 1). The 5′ pUC18 and 3′ pUC19 clones were amplified by growth in E. coli, and the coding regions were digested from the isolated plasmids as HindIII-EcoRI fragments. These fragments were ligated, digested with HindIII, and the complete ORF with HindIII termini was cloned into HindIII-digested YEp52 (pYGAL/PSSC: Figure 1). The complete ORF from pYGAL/PSSC was purified by preparative agarose gel electropholesis for cloning and expression in the alpha factor promoter system, as described in the following Examples.

EXAMPLE 2

Preparation of an alpha mating factor expression vector

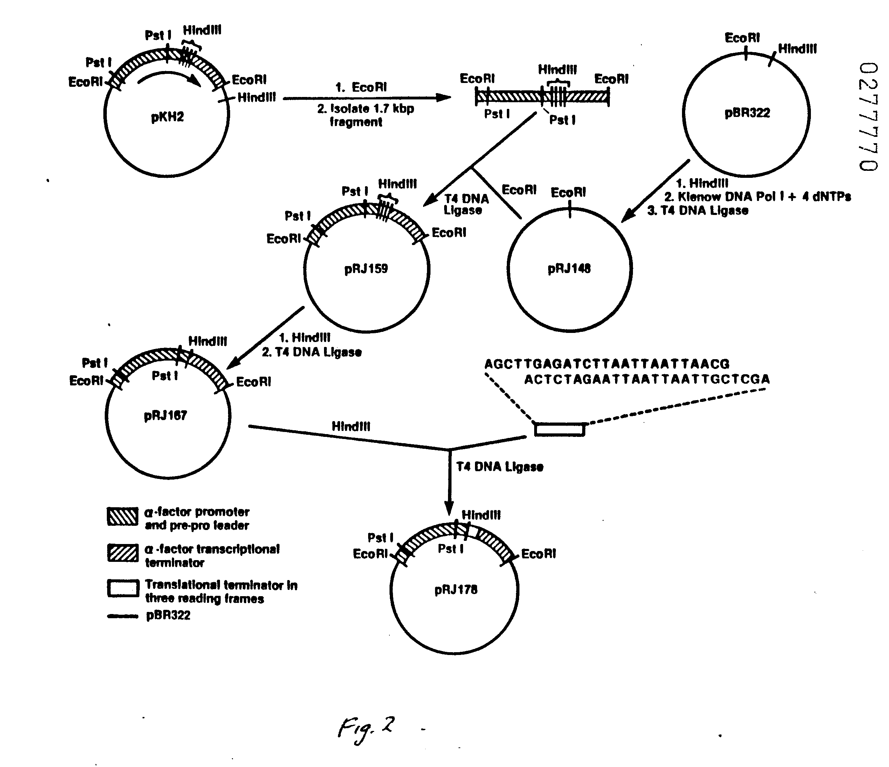

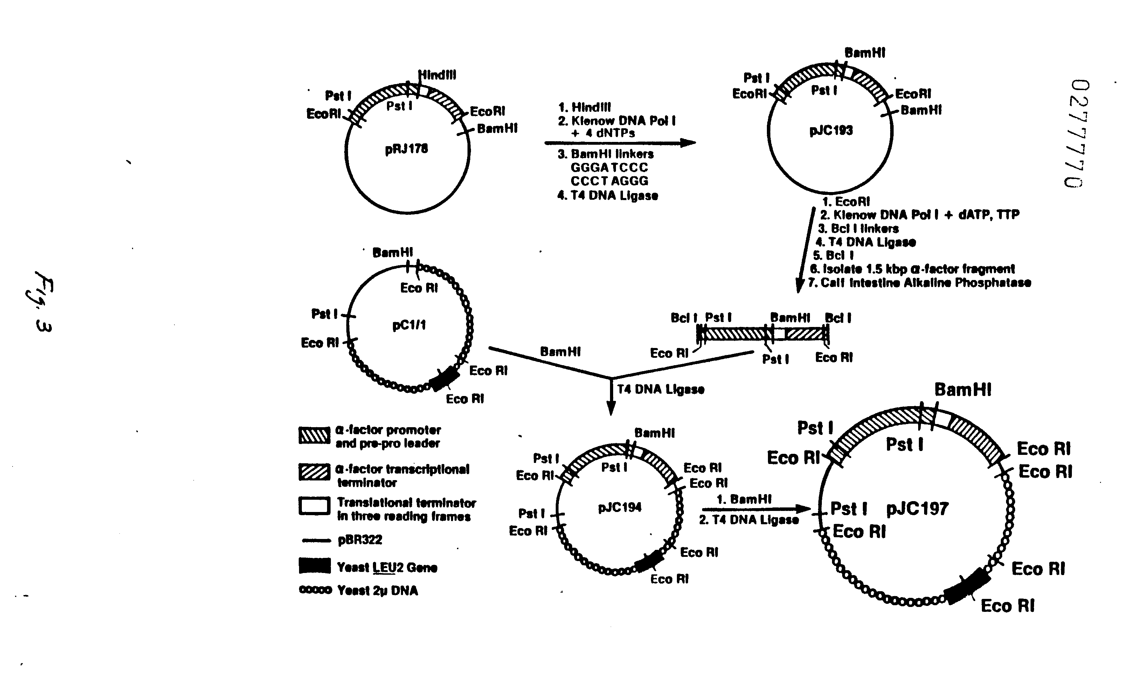

Plasmid pKH2 wherein the alpha mating factor gene MFα1 is cloned onto a plasmid from S. cerevisiae genomic DNA is prepared according to the procedures of Kurjan et al. [Cell 30: 933 (1982)] and Singh et al. [Nucleic Acids Res. 11: 4049 (1983)]. Plasmid pKH2 was digested with EcoRI and the 1.7 kbp fragment bearing the alpha mating factor gene was purified by preparative agarose gel electrophoresis (Figure 2, top) Plasmid pRJ148 (a modified pBR322 lacking the HindIII site) was digested with EcoRI and ligated with the 1.7 kbp fragment to yield the plasmid pRJ159 (figure 2, middle). This DNA was digested with HindIII and self-ligated to form plasmid pRJ167, which has a unique HindIII site (Figure 2, bottom). Plasmid pRJ167 was digested with HindIII and modified by the insertion of a synthetic oligonucleotide adaptor to yield a new plasmid (pRJ178) containing a unique HindIII site that is located on the 3′ side of the promoter and pre-pro-leader and on the 5′ side of the translational termination signals in all three reading frames (Figure 2, bottom). The HindIII site was converted to a BamHI site by digestion with HindIII, treatment with the Klenow fragment of DNA polymerase I, addition of BamHI linkers and self-ligation to form plasmid pJC193 (Figure 3, top). This plasmid was digested with EcoRI, flush-ended with the Klenow fragment of DNA polymerase I, modified by the addition of BclI linkers, digested with BclI, and the 1.5 kbp fragment bearing the alpha mating factor gene isolated by preparative gel electrophoresis (Figure 3, middle). This resulting BclI fragment then was inserted into the unique BamHI site of pCI/1, destroying the original BamHI site in the process (plasmid pJC194; Figure 3, bottom). This DNA was digested with BamHI and self-ligated to remove excess BamHI linkers (Figure 3, bottom). As a result, a new alpha mating factor expression plasmid (pJC197) was created which has the following salient features: (1) E. coli-derived sequences for the selection (bla gene) and amplification (ori) of the plasmid in E. coli, (2) S. cerevisiae-derived sequences for the selection (LEU2) and amplification (2-micron DNA) of the plasmid in S. cerevisiae, (3) yeast alpha mating factor promoter, (4) yeast alpha mating factor pre-leader for directing the translational product into the rough endoplasmic reticulum, (5) yeast alpha mating factor pro-leader which encodes N-glycosylation signal sequences and which is cleaved by the KEX2 and STE13 proteases (gene products) [Julius et al., Cell 32: 839 (1983); Julius et al., Cell 37: 1075 (1984)], (6) a unique BamHI site for the cloning of a foreign polypeptide to be expressed, (7) a translational termination sequence in all three reading frames, and (8) a yeast transcriptional termination sequence.

EXAMPLE 3

Preparation of an expression vector with the alpha mating factor promoter without the leader sequence.

Plasmid pJC197 containing the MFα1 promoter and structural gene was digested with PstI, and the 0.8 kbp fragment containing both the promoter and pre-pro-leader sequences was purified by preparative agarose gel electrophoresis. It then was digested with Bal31 under conditions where 30-40 bp were removed from each end. The DNA was blunt-ended with T4 DNA polymerase and ligated to a synthetic BamHI octameric linker of the structure:

CGGATCCG

GCCTAGGC.

The DNA was digested with BamHI and cloned into the BamHI site of pBR322 thus creating pBRαF; transformants of E. coli strain HB101 were screened with two synthetic oligonucleotides that had been end-labelled with γ[$^{32}$P]ATP in the presence of T4 polynucleotide kinase.

CTATCAATTTCATACACAATA - oligonucleotide "a", derived from the 5′ noncoding sequence immediately flanking the coding sequence.

ACCAAAAGAATGAGATTTCC - oligonucleotide "b", derived from the immediate 5′ end of the coding sequence.

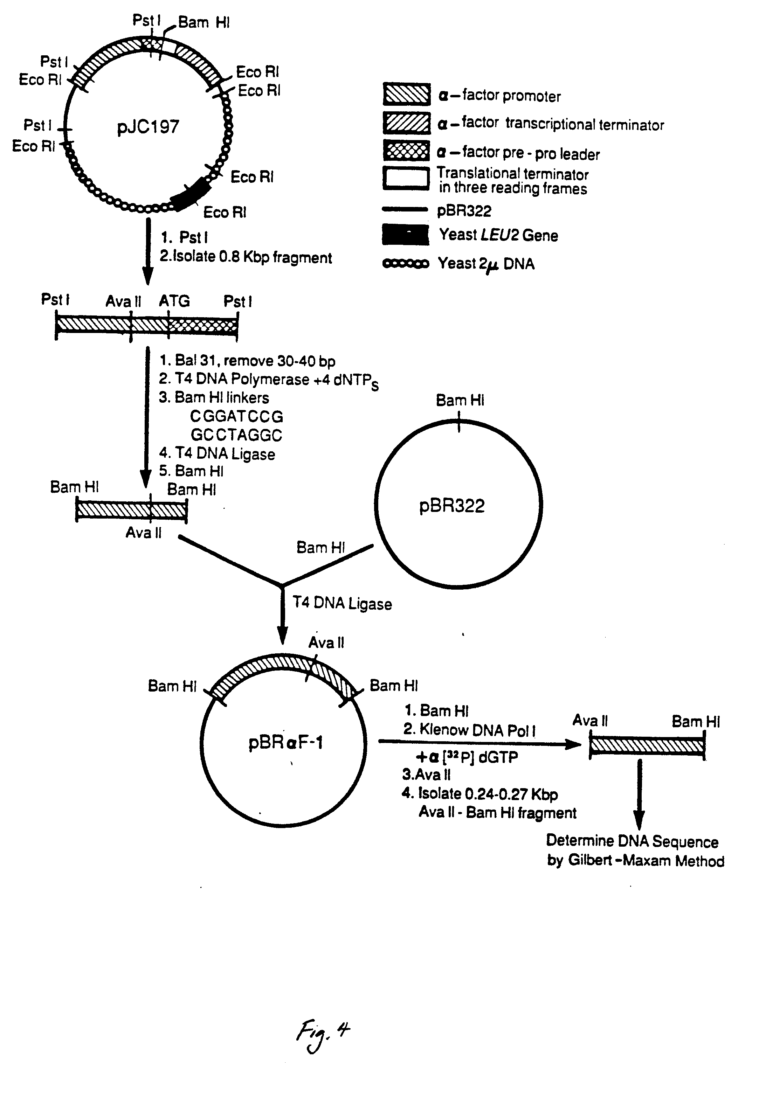

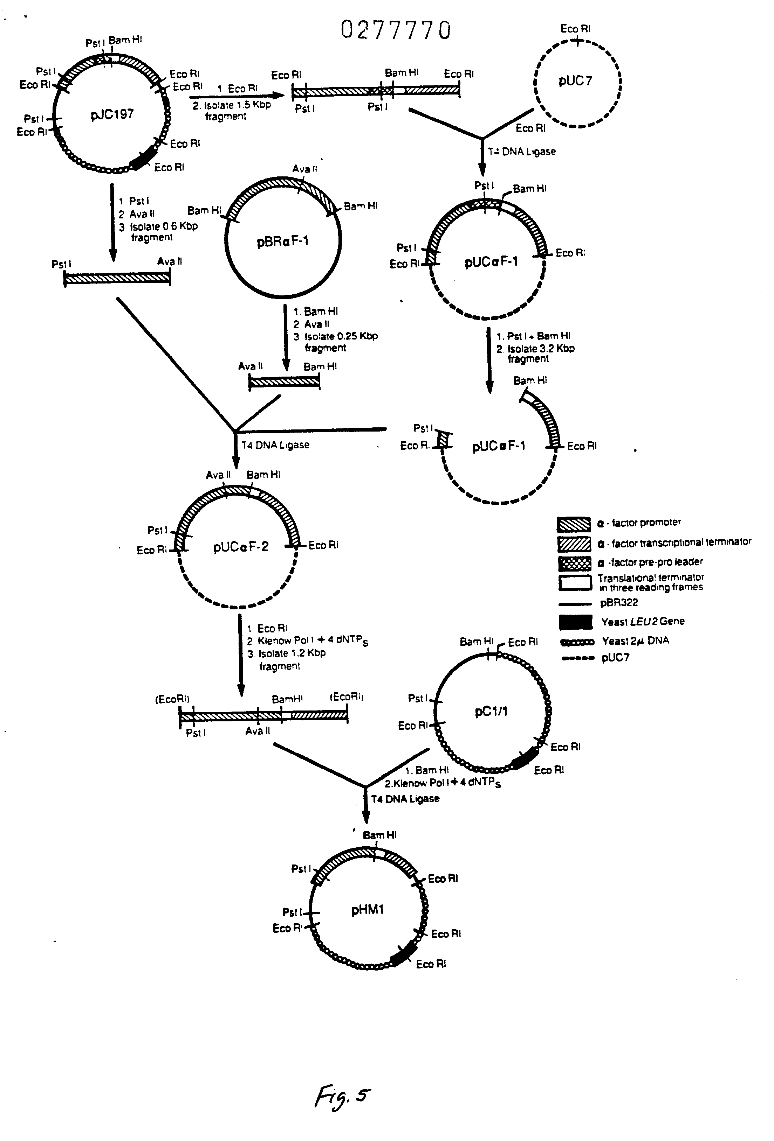

Colonies which hybridized only with oligonucleotide "a" were selected for further analysis. DNA was extracted from 3 such colonies, digested with BamHI, labelled with α[$^{32}$P]dGTP in the presence of the Klenow fragment of DNA polymerase I, and digested with AvaII. Several 0.24-0.27 kbp fragments were purified by preparative gel electrophoresis and sequenced by the Maxam-Gilbert method to verify the absence of the 5′ leader region of the coding sequence (Figure 4, bottom). From a candidate clone designated pBRαF-1, a larger amount of the 0.25 kbp BamHI-AvaII fragment was purified by preparative gel electrophoresis (Figure 5, top middle). The 1.5 kbp fragment of EcoRI-digested pJC197 was purified by preparative gel electrophoresis and cloned into the EcoRI site of pUC7. The resulting transformants were designated pUCαF-1 (Figure 5, top right). pJC197 (Figure 5, top left) was digested with PstI, and the 0.8 kbp MFα1 promoter fragment was isolated. This fragment then was digested with AvaII, and the 0.6 kbp fragment was purified by preparative gel electrophoresis. pUCαF-1 was digested with PstI and BamHI, and the 3.2 kbp fragment was purified by preparative agarose gel electrophoresis and ligated to the previously isolated 0.25 and 0.6 kbp fragments of the MFα1 promoter and used to transform E. coli. The resulting vector pUCαF-2 was digested with EcoRI. The 1.2 kbp fragment was blunt-ended with the Klenow fragment of DNA polymerase I, purified by preparative agarose gel electrophoresis, and ligated into the blunt-ended BamHI site of pCl/1. The resulting vector, pHM1 (Figure 5, bottom), has the following salient features:

1) E. coli-derived sequences for the selection (bla gene) and amplification (ori) of the plasmid in E. coli.

2) S. cerevisiae-derived sequences for the selection (LEU2) and amplification (2-micron DNA) of the plasmid in S. cerevisiae.

3) yeast alpha mating factor promoter.

4) a unique BamHI cloning site for the cloning of a foreign polypeptide to be expressed.

5) a translational termination sequence in all three reading frames.

6) a yeast transcriptional termination sequence.

## EXAMPLE 4

### Expression of HBV PreS1/PreS2/S in the regulatable expression vector pHM1

The alpha mating factor promoter is active only in cells which are phenotypically $\alpha$ [Brake et al., Mol. Cell Biol. 3: 1340 (1983)]. There are 4 loci in S. cerevisiae, known as SIR, which synthesize proteins required for the repression of other normally silent copies of a and $\alpha$ information (Rine et al., op. cit.) Strain JRY188 cells (MAT$\alpha$, sir3-8, leu2-3, leu2-112, trp1, ura3-52, his4) contain a ts lesion in the SIR3 gene product. As a result, JRY188 cells grown at 35°C are phenotypically a/$\alpha$ and the alpha mating factor promoter is not active; on the other hand, cells grown at 23°C are phenotypically $\alpha$ and thus capable of expressing genes directed by the alpha mating factor promoter [Brake et al., Proc. Natl. Acad. Sci. USA 81: 4642 (1984)].

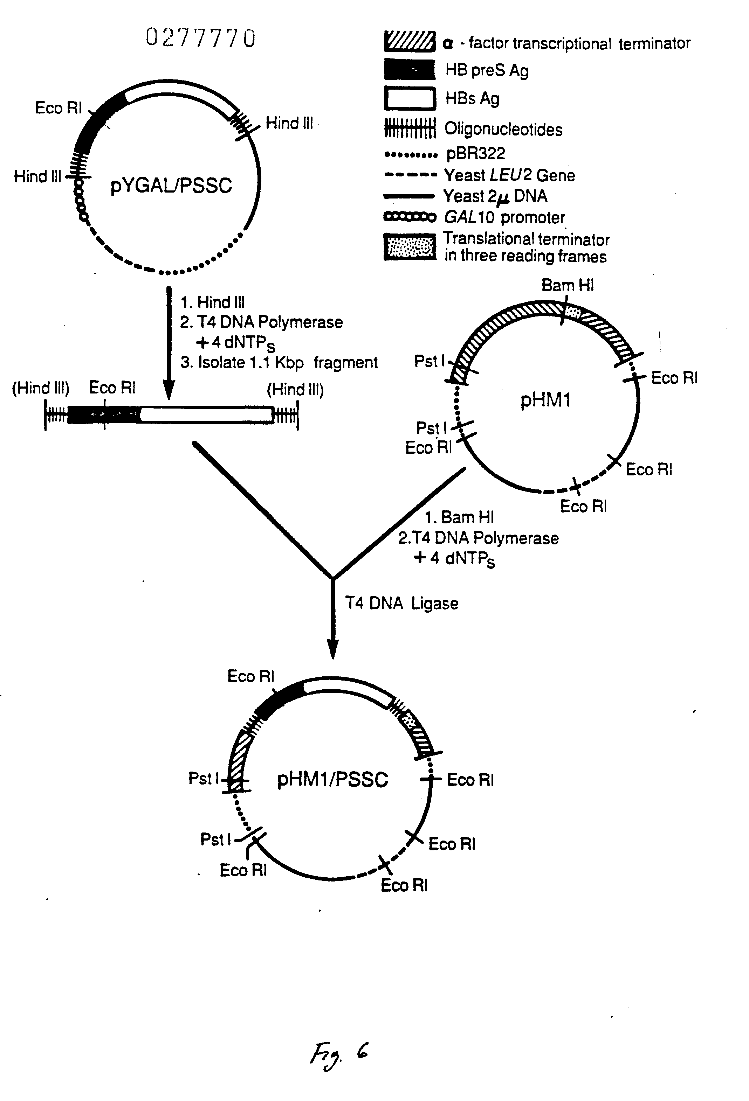

The vector pYGAL/PSSC of Example 1 was digested with HindIII, and the 1.1 kbp preS1/preS2/S ORF was flush-ended with the Klenow fragment of DNA polymerase I, purified by preparative agarose gel electrophoresis, and cloned into the BamHI site of pHM1 that had been flush-ended with T4 DNA polymerase. The resultant plasmid pHM1/PSSC (Figure 6) was introduced into strain JRY188 cells and transformed clones (JRY188/pHM1/PSSC) were selected and amplified. Growth of the cells at 35°C resulted in a complete lack of detectable synthesis of preS1/preS2/S. Cells initially were grown to a saturation density at 35°C for 24 hours in a volume of 5 ml. The culture was diluted 1:20 into 5 ml of fresh medium and grown for an additional 48 hours at 23°C. Expression of the desired antigen was verified by the detection of HBsAg by AUSRIA® (Abbott) reactivity, polymerized human albumin binding activity [Machida et al., Gastroenterology 86: 910 (1984)], and the presence of p39 in immunoblots which were developed using convalescent human serum and radiolabelled Staphylococcus aureus protein A. Evidence for the particulate form of HBsAg is the detectable expression of antigen by AUSRIA® reactivity, an assay that detects only the particulate form of HBsAg.

Samples of JRY 188/pHM1/PSSC have been deposited as ATCC NO        on or before the filing of the present application at the American Type Culture Collection, 12301 Parklawn Drive, Rockville, Md. 20852. JRY 188 is a strain of the common yeast Saccharomyces cerevisiae.

While the foregoing specification teaches the principles of the present invention, with examples provided for the purpose of illustration, it will be understood that the practice of the invention encompasses all of the usual variations, adaptations, modifications, deletions or additions of procedures and protocols described herein, as come within the scope of the following claims.

## Claims

1. A plasmid expression vector comprising an E. coli-derived DNA sequence for selection and propagation in E. coli, a yeast-derived DNA sequence for selection and propagation of the plasmid in yeast cells of a species within the Families Cryptococcaceae or Saccharomycetaeceae, a promoter from the yeast alpha mating factor gene without the coding sequences for the yeast alpha mating factor pre-pro-leader, a translational termination sequence for at least one reading frame, a yeast transcriptional termination sequence, and a unique restriction endonuclease cloning site for insertion of a sequence of DNA between the promoter and the translational termination sequence, said DNA coding for a heterologous polypeptide.

2. A plasmid expression vector according to Claim 1 wherein the heterologous polypeptide is hepatitis B virus preS1/preS2/S polypeptide or portion thereof capable of reacting immunochemically with antibodies specific for hepatitis B virus preS1/preS2/S polypeptide.

3. pHM1.

4. pHM1/PSSC.

5. A culture of yeast cells comprising cells of a species within the Families Saccharomycetaeceae or Cryptococcaceae with a ts lesion in the gene product of one of its SIR genes or a functionally analogous gene, transformed by a plasmid expression vector comprising an E. coli-derived DNA sequence for selection and propagation in E. coli, and a yeast-derived DNA sequence for selection and propagation of the plasmid in yeast cells of a species within the Families Cryptococcaceae or Saccharomycetaeceae, a promoter from the yeast alpha mating factor gene without the coding sequences for yeast alpha mating factor pre-pro-leader, a translational termination sequence for at least one reading frame, a yeast transcriptional termination sequence, and a unique restriction endonuclease cloning site for insertion of a sequence of DNA between the promoter and the translational terminator, said DNA coding for a heterologous polypeptide sequence, said cloning site having inserted thereto a sequence of DNA coding for a heterologous protein, said yeast cells expressing said heterologous protein.

6. JRY188/pHM1/PSSC.

7. A process for obtaining a predetermined polypeptide in a host cell of a species within Families Saccharomycetaeceae or Cryptococcaceae comprising

(a) transforming a culture of yeast cells of one or more species within said Families with a plasmid expression vector comprising an E. coli-derived DNA sequence for selection and propagation in E. coli, and a yeast-derived DNA sequence for selection and propagation of the plasmid in yeast cells of a species within the Families Cryptococcaceae or Saccharomycetaeceae, a promoter from the yeast alpha mating factor gene without the coding sequences for yeast alpha mating factor pre-pro-leader, a translational termination sequence for at least one reading frame, a yeast transcriptional termination sequence, and a unique restriction endonuclease cloning site for insertion of a sequence of DNA between the promoter and the translational termination sequence, said DNA coding for a heterologous polypeptide sequence, said cloning site having inserted thereto a sequence of DNA coding for a heterologous protein, said yeast cells expressing said heterologous protein;

(b) culturing the transformed cells of step (a); and

(c) recovering said heterologous protein from the cultured transformed cells of step (b).

8. The process of Claim 7 wherein the species is of the genus Saccharomyces.

9. The process of Claim 8 wherein the species is S. cerevisiae.

10. The process of Claim 7, 8 or 9 wherein the heterologous protein is hepatitis B virus preS1/preS2/S polypeptide, or portion thereof capable of reacting immunochemically with antibodies specific for hepatitis B virus preS1/preS2/S polypeptide.

11. A process for obtaining recombinant hepatitis B virus preS1/preS2/S polypeptide comprising the steps of

(a) transforming a culture of JRY188 cells of the species S. cerevisiae with pHM1/PSSC, to yield JRY188/pHM1/PSSC expressing hepatitis B virus preS1/preS2/3 polypeptide;

(b) culturing JRY188/pHM1/PSSC of step (a); and

(c) recovering hepatitis B virus preS1/preS2/S protein as product from the cultured transformed cells of step (b).

12. The product of the process of Claim 11.

0277770

Fig. 1

Pst I  HindIII
Pst I
Pst I
EcoRI
HindIII

pKH2

1. EcoRI
2. Isolate 1.7 kbp fragment

EcoRI        HindIII        EcoRI

Pst I        Pst I

EcoRI        HindIII

pBR322

T4 DNA Ligase

EcoRI

Pst I        HindIII
Pst I
EcoRI        EcoRI

pRJ159

EcoRI        EcoRI

1. HindIII
2. Klenow DNA Pol I + 4 dNTPs
3. T4 DNA Ligase

pRJ148

1. HindIII
2. T4 DNA Ligase

Pst I
HindIII
Pst I
EcoRI        Pst I        EcoRI

pRJ167

HindIII

AGCTTGAGATCTTAATTAATTAACG
ACTCTAGAATTAATTAATTGCTCGA

T4 DNA Ligase

SNW  α-factor promoter and pre-pro leader

α-factor transcriptional terminator

Translational terminator in three reading frames

pBR322

HindIII
Pst I
EcoRI        Pst I        EcoRI

pRJ178

Fig. 2

0277770

0277770

Fig. 3

0277770

Fig. 4

Fig. 5

0277770

**Legend:**
- α - factor transcriptional terminator
- HB preS Ag
- HBs Ag
- Oligonucleotides
- pBR322
- Yeast *LEU*2 Gene
- Yeast 2μ DNA
- *GAL*10 promoter
- Translational terminator in three reading frames

pYGAL/PSSC

Eco RI, Hind III, Hind III

1. Hind III
2. T4 DNA Polymerase + 4 dNTP$_S$
3. Isolate 1.1 Kbp fragment

(Hind III) Eco RI (Hind III)

pHM1

Bam HI, Pst I, Pst I, Eco RI, Eco RI, Eco RI, Eco RI

1. Bam HI
2. T4 DNA Polymerase + 4 dNTP$_S$

T4 DNA Ligase

pHM1/PSSC

Eco RI, Pst I, Pst I, Eco RI, Eco RI, Eco RI, Eco RI

*Fig. 6*

European Patent Office

# EUROPEAN SEARCH REPORT

Application number

## DOCUMENTS CONSIDERED TO BE RELEVANT

EP 88300721.3

| Category | Citation of document with indication. where appropriate, of relevant passages | Relevant to claim | CLASSIFICATION OF THE APPLICATION (Int. Cl.4) |
|---|---|---|---|
| D,A | US - A - 4 546 082 (KURJAN et al.)<br>* Abstract *<br>-- | 1 | C 12 N 15/00<br>C 12 N 1/16<br>C 12 P 21/00 |
| P,A | EP - A1 - 0 211 767 (MERCK & CO. INC.)<br>* Example 1; claims 1-3,10-12 *<br>-- | 1,2,11, 12 | /(C 12 P 21/00;<br>C 12 R 1:85)<br>(C 12 P 21/00;<br>C 12 R 1:865) |
| D,A | PROCEEDINGS OF THE NATIONAL ACADEMY OF SCIENCES OF THE UNITED STATES OF AMERICA, vol. 80, no. 23, December 1983 (Baltimore, USA)<br>S.D. EMR et al.: "An MF$\alpha$1-SUC$_2$ ($\alpha$-factor-invertase)gene fusion for study of protein localization and gene expression in yeast" pages 7080-7084<br>* Totality *<br>-- | 1 | |
| | | | TECHNICAL FIELDS SEARCHED (Int. Cl.4) |
| D,A | PROCEEDINGS OF THE NATIONAL ACADEMY OF SCIENCES OF THE UNITED STATES OF AMERICA, vol. 81, no. 15, August 1984 (Baltimore, USA)<br>A.J. BRAKE et al.: "$\alpha$-Factor-directed synthesis and secretion of mature foreign proteins in saccharomyces cerevisiae" pages 4642-4646<br>* Totality *<br>-- | 1 | C 12 N<br>C 12 P |

The present search report has been drawn up for all claims

| Place of search | Date of completion of the search | Examiner |
|---|---|---|
| VIENNA | 10-05-1988 | WOLF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82

**European Patent Office**

**EUROPEAN SEARCH REPORT**

Application number

| | DOCUMENTS CONSIDERED TO BE RELEVANT | | EP 88300721.3 |

| Category | Citation of document with indication, where appropriate, of relevant passages | Relevant to claim | CLASSIFICATION OF THE APPLICATION (Int. Cl.4) |
|---|---|---|---|
| D,A | PROCEEDINGS OF THE NATIONAL ACADEMY OF SCIENCES OF THE UNITED STATES OF AMERICA, vol. 81, no. 17, September 1984 (Baltimore, USA)<br><br>G.A. BITTER et al.: "Secretion of foreign proteins from Saccharomyces cerevisiae directed by α-factor gene fusions" pages 5330-5334<br><br>    * Totality *<br><br>    -- | 1 | |
| D,A | NUCLEIC ACIDS RESEARCH, vol. 12, no. 23, December 11, 1984 (London)<br><br>A. SINGH et al.: "Synthesis, secretion and processing of α-factor-interferon fusion proteins in yeast" pages 8927-8938<br><br>    * Totality *<br><br>    -- | 1 | |
| D,A | J.N. STRATHERN et al.: "The Molecular Biology of the Yeast Saccharomyces", Cold Spring Harbor Laboratory, 1981 (New York)<br><br>I. HERSKOWITZ et al.: "Control of Cell Type in Saccharomyces cerevisiae: Mating type and Mating-type Interconversion" pages 181-209<br><br>    * Totality *<br><br>    -- | | TECHNICAL FIELDS SEARCHED (Int. Cl.4) |

The present search report has been drawn up for all claims

| Place of search | Date of completion of the search | Examiner |
|---|---|---|
| VIENNA | 10-05-1988 | WOLF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82

. Application number

| European Patent Office | EUROPEAN SEARCH REPORT | |
|---|---|---|

| DOCUMENTS CONSIDERED TO BE RELEVANT | | | EP 88300721.3 |
|---|---|---|---|
| Category | Citation of document with indication, where appropriate, of relevant passages | Relevant to claim | CLASSIFICATION OF THE APPLICATION (Int. Cl 4) |
| D,A | H.L. ROMAN et al.: "Annual Review of Genetics", vol. 16, 1982 (Palo Alto, USA)<br><br>K.A. NASMYTH: "Molecular Genetics of Yeast Mating Type" pages 439-500<br><br>    * Totality *<br><br>---- | | |
| | | | TECHNICAL FIELDS SEARCHED (Int. Cl.4) |

The present search report has been drawn up for all claims

| Place of search | Date of completion of the search | Examiner |
|---|---|---|
| VIENNA | 10-05-1988 | WOLF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82